# EUROPÄISCHE PATENTSCHRIFT

(11) **EP 0 820 975 B1**
(45) Veröffentlichungstag und Bekanntmachung des Hinweises auf die Patenterteilung: **07.03.2001**
(21) Anmeldenummer: 97111108.3
(22) Anmeldetag: 03.07.1997
(51) Int. Cl.: C07C 43/04, C07C 11/02, C07C 41/06, C07C 2/08

(54) **Verfahren zur Herstellung von Alkyltert-butylethern und Di-n-buten aus Feldbutanen**
Process for the preparation of alkyl tert-butyl ethers and di-n-butene from field butanes
Procédé pour la préparation d'éthers alkyls tertiobutyls à partir de butanes naturels

(30) Priorität: 24.07.1996 DE 19629904
(43) Veröffentlichungstag der Anmeldung: 28.01.1998
(73) Patentinhaber: Degussa-Hüls Aktiengesellschaft, 60287 Frankfurt am Main (DE)
(72) Erfinder: Nierlich, Franz, Dr., 45768 Marl (DE); Olbrich, Paul, Dr., 45721 Haltern (DE); Droste, Wilhelm, Dr., 45770 Marl (DE); Müller, Richard, Dr., 45770 Marl (DE); Toetsch, Walter, Dr., 45770 Marl (DE)

(56) Entgegenhaltungen:
- WO-A-90/11268
- FR-A- 2 594 139
- P. R. SARATHY ET AL: "Etherify field butanes. Part 2" HYDROCARBON PROCESSING., Februar 1993, HOUSTON US, Seite 43-46,48,50-51 XP000343972

## Beschreibung

Die Erfindung betrifft ein Verfahren zur Herstellung von Alkyl-tert.butylether (in der Folge kurz RTBE genannt, wobei R für Alkyl steht) und von Di-n-buten in einer Koppelproduktion aus Feldbutanen, wobei man das iso-Butan in Alkyl-tert.butylether und das n- Butan in Di-n-buten umwandelt und das Mengenverhältnis dieser beiden Produkte regeln kann, indem man das Mengenverhältnis von n-Butan zu iso-Butan durch Isomerisierung entsprechend einstellt.

RTBE werden als Zusatz zu Fahrbenzin zur Erhöhung der Octanzahl verwendet. Man stellt sie durch Anlagerung von Alkanolen an iso-Buten her, die auch als Veretherung bezeichnet wird. Das iso-Buten kann aus vier verschiedenen Quellen stammen: Aus Steamcrackern, Propylenoxid-Anlagen, Erdölraffinerien (d.h. FC-Crackern) und Anlagen zur Dehydrierung von iso-Butan (vgl. R.A.Pogliano et al., Dehydrogenation-Based Ether Production - Adding Value to LPG and Gas Condensate. 1996 Petrochemical Review, DeWitt & Company, Houston Texas). Bei den ersten drei Quellen fällt das iso-Buten als Bestandteil des C₄-Schnitts, also als unmittelbares Nebenprodukt an. Bei der Dehydrierung von iso-Butan ist iso-Buten oft ein mittelbares Nebenprodukt solcher Anlagen, denn der Ausgangsstoff iso-Butan wird ebenfalls als unmittelbares Nebenprodukt in Steamcrackern und Erdölraffinerien oder durch Isomerisierung von n-Butan gewonnen. das seinerseits ein Nebenprodukt in Steamcrackern und Erdölraffinerien ist. Die derzeitige Weltproduktion von RTBE beträgt rund 25 Millionen t/Jahr, mit steigender Tendenz. Der Anfall an Butanen und Butenen als Nebenprodukte in einem bestimmten Cracker oder einer bestimmten Erdölraffinerie ist zu klein, als daß man die "Economies of Scale". die in dem RTBE-Verfahren stecken, voll ausnutzen könnte. Man müßte also iso-Buten und/oder iso-Butan (zum Dehydrieren) aus Crackern und/oder Raffinerien sammeln, um eine RTBE-Anlage mit optimaler Kapazität betreiben zu können. Alternativ könnte man genügend C₄-Schnitt aus solchen Anlagen sammeln und diesen vor Ort auf iso-Buten und iso-Butan aufarbeiten. Beiden Varianten, und insbesondere der zweiten, steht aber entgegen, daß der Transport von Flüssiggasen teuer ist, nicht zuletzt wegen der aufwendigen Sicherheitsmaßnahmen.

Als Dibuten bezeichnet man das Isomerengemisch; das neben höheren Butenoligomeren durch Dimerisierung und/oder Co-Dimerisierung von Butenen, d.h. von n-Buten und/oder iso-Buten, bei der Oligomerisierung von Butenen entsteht. Als Di-n-buten bezeichnet man das Dimerisierungsprodukt von n-Buten, d.h. 1-Buten und/oder 2-Buten. Wesentliche Bestandteile des Di-n-butens sind 3-Methyl-2-hepten, 3,4-Dimethyl-2hexen und in untergeordnetem Maße n-Octene. Di-iso-buten ist das Isomerengemisch, das durch Dimerisierung von iso-Buten entsteht. Di-isobuten ist stärker verzweigt als Dibuten und dieses wiederum stärker verzweigt als Di-n-buten.

Dibuten, Di-n-buten und Di-iso-buten sind Ausgangsstoffe für die Herstellung von isomeren Nonanolen durch Hydroformylierung und Hydrierung der so entstehenden C₉-Aldehyde. Ester dieser Nonanole. insbesondere die Phthalsäureester, sind Weichmacher, die in bedeutendem Umfang hergestellt und vor allem für Polyvinylchlorid verwendet werden. Nonanole aus Di-n-buten sind in höherem Maße geradkettig als Nonanole aus Dibuten, die wiederum weniger verzweigt sind als Nonanole aus Di-iso-buten. Ester von Nonanolen aus Di-n-buten haben anwendungstechnische Vorteile gegenüber Estern aus anderen Nonanolen und sind daher besonders gefragt.

Man gewinnt n-Buten für die Dimerisierung. ebenso wie iso-Buten, beispielsweise aus C₄-Schnitten, wie sie in Steamcrackern oder FC-Crakkern anfallen. Die C₄-Schnitte werden in der Regel aufgearbeitet, indem man zunächst 1.3-Butadien durch eine selektive Wäsche. z.B. mit N-Methylpyrrolidon, abtrennt. iso-Buten ist ein erwünschter und besonders wertvoller Bestandteil des C₄-Schnitts, weil es sich. allein oder im Gemisch mit anderen C₄-Kohlenwasserstoffen. zu begehrten Produkten chemisch umsetzen läßt, z.B. mit iso-Butan zu hochoctanigem iso-Octan oder mit Methanol zu Methyl-tert.butylether (MTBE). dem wichtigsten RTBE. Nach der Umsetzung des iso-Butens bleiben die n-Butene sowie n-Butan und iso-Butan zurück. Der Anteil des n-Butens an den Spaltprodukten der Steamcracker bzw. der Erdölraffinerien ist jedoch verhältnismäßig gering. Bei Steamcrackern liegt er in der Größenordnung von knapp 10 Gewichtsprozent, bezogen auf das hauptsächliche Zielprodukt Ethylen. Ein Steamcracker mit der respektablen Kapazität von 600.000 t/Jahr Ethylen liefert also nur rund 60.000 t/Jahr n-Buten. Man könnte zwar dessen Menge (und die der iso-Butene) erhöhen, indem man die rund 15.000 t/Jahr n- und iso-Butan dehydriert, die neben den n-Butenen anfallen. Das empfiehlt sich jedoch nicht, weil Dehydrieranlagen hohe Investitionskosten erfordern und für eine so kleine Kapazität unwirtschaftlich sind.

iso-Buten ist, wie gesagt, ein gefragtes Crackprodukt und steht daher für die Isomerisierung zu n-Buten in der Regel nicht zur Verfügung. Die Menge an n-Butenen, die ein Steamcracker oder eine Erdölraffinerie unmittelbar erzeugt, reicht jedoch nicht aus, um genügend Di-n-buten für eine Nonanol-Anlage zu erzeugen, deren Kapazität so groß ist, daß sie mit den bestehenden großen Anlagen zur Herstellung bedeutender Weichmacheralkohole, wie 2-Ethylhexanol, wirtschaftlich konkurrieren könnte. Propylenoxid-Anlagen sind, wie schon gesagt. noch weniger ergiebig. Man müßte also n-Butene aus verschiedenen Steamcrackern, Raffinerien oder Propylenoxid-Anlagen sammeln (oder C₄-Schnitt aus verschiedenen Quellen auf n-Buten aufarbeiten) und das n-Buten vereint oligomerisieren, um den Bedarf einer hinreichend großen, wirtschaftlichen Nonanol-Anlage an Dibuten zu decken. Der Transport von Flüssiggasen ist jedoch teuer, wie bereits erwähnt.

Es wäre daher erwünscht, wenn man n-Buten und iso-Buten an nur einem Standort ohne Transport über größere Entfernungen in Mengen zur Verfügung stellen könnte, wie sie in einer Koppelproduktion für den Betrieb einer großen, wirtschaftlich vorteilhaften Anlage zur Herstellung von Di-n-buten. beispielsweise mit einer Kapazität von 200.000 bis 800.000 t/Jahr, und einer ebensolchen Anlage zur Herstellung von MTBE, z.B. mit einer Kapazität von 300.000 bis 800.000 t/Jahr, erforderlich sind. Es wäre weiterhin erwünscht, den Verbund dieser Anlagen so zu gestalten, daß man das Mengenverhältnis von n-Buten zu iso-Buten den gewünschten Mengen an Di-n-buten und MTBE entsprechend einstellen kann.

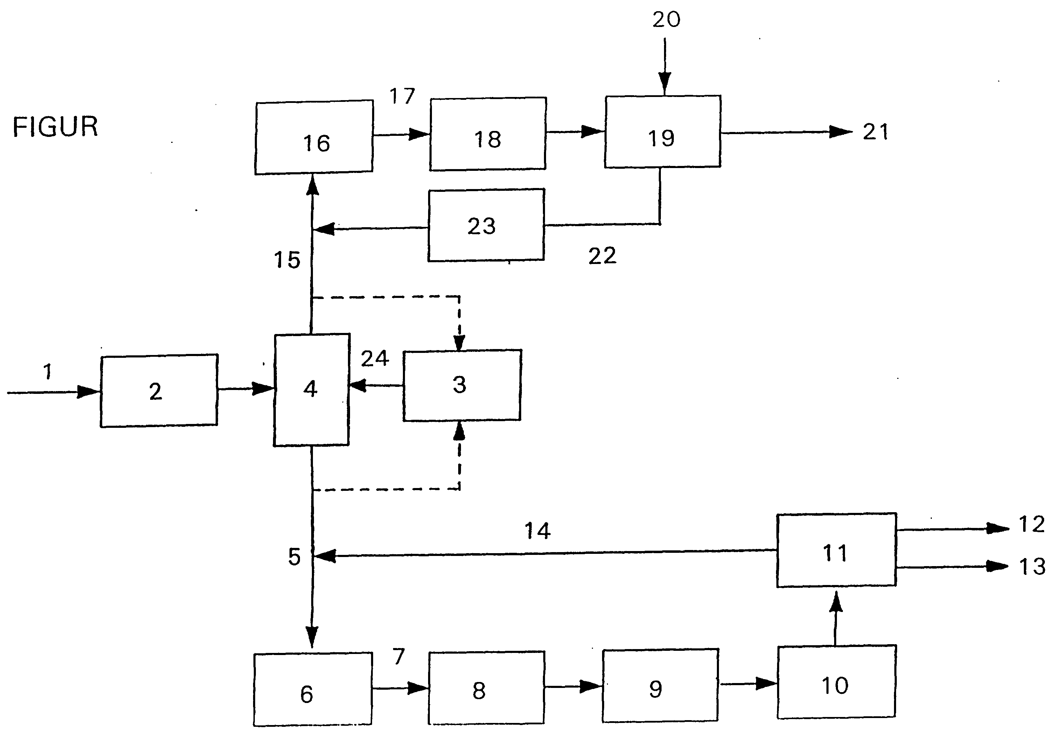

Eine Anlage, die diesen Erfordernissen entspricht, ist mit ihren wesentlichen sowie mit fakultativen Merkmalen in der beigefügten Figur als Blockschema dargestellt.

Die Erfindung ist ein Verfahren zur Herstellung von Di-n-buten und Alkyl-tert.butylethern in einer Koppelproduktion aus Feldbutanen. bei dem man
(a) die Feldbutane 1 in der Trennstufe 4 in n-Butan und iso-Butan trennt,
(b) das n-Butan 5 in einer Dehydrierungsstufe 6 zu einem n-Buten enthaltenden Dehydrierungsgemisch 7 dehydriert, das n-Buten in der Oligomerisierungsstufe 10 zu einem Oligomerengemisch 11 oligomerisiert und aus diesem Di-n-buten 12 abtrennt, und
(c) das iso-Butan 15 in der Dehydrierungsstufe 16 zu einem iso-Buten enthaltenden Dehydrierungsgemisch 17 dehydriert und das iso-Buten in der Veretherungsstufe 19 mit einem Alkanol 20 zu einem Alkyl-tert.butylether 21 umsetzt.

Eine bevorzugte Ausführungsform des Verfahrens ist dadurch gekennzeichnet, daß man die Feldbutane 1 vor dem Eintritt in die Trennstufe 4 in der Hydrierstufe 2 Hydrierungsbedingungen unterwirft und der Trennstufe 4 eine Isomerisierungsstufe 3 zuordnet, durch die das Mengenverhältnis von n-Butan zu iso-Butan nach Maßgabe des gewünschten Mengenverhältnisses von Di-n-buten zu Alkyl-tert.butylether eingestellt werden kann.

Das Verfahren der Erfindung zeichnet sich durch hohe Flexibilität aus, denn man kann innerhalb der Grenzen, die durch die Kapazitäten der Di-n-buten-Anlage und der RTBE-Anlage gezogen sind. die Mengen an Di-n-buten und an RTBE den Markterfordernissen entsprechend variieren.

Als Feldbutane bezeichnet man die C₄-Fraktion der "feuchten" Anteile des Erdgases sowie der Erdölbegleitgase, die durch Trocknung und Abkühlung auf etwa -30°C in flüssiger Form aus den Gasen abgetrennt werden. Durch Tieftemperaturdestillation gewinnt man daraus die Feldbutane, deren Zusammensetzung je nach Lagerstätte schwankt, die jedoch im allgemeinen etwa 30% iso-Butan und etwa 65% n-Butan enthalten. Weitere Bestandteile sind in der Regel etwa 2 % C_{<4}-Kohlenwasserstoffe und etwa 3% C_{>4}-Kohlenwasserstoffe. Feldbutane können ohne Auftrennung als Einsatzstoffe in Steamcrackern oder als Zusatz zum Fahrbenzin verwendet werden. Sie lassen sich durch fraktionierte Destillation in n-Butan und iso-Butan zerlegen. Iso-Butan wird z.B. in bedeutendem Umfang für die Herstellung von Propylenoxid durch Co-Oxidation von Propylen und iso-Butan sowie als Alkylierungsmittel verwendet, mit dem n-Buten bzw. iso-Buten zu iso-Octan alkyliert wird, das wegen seiner hohen Octanzahl als Zusatz zum Fahrbenzin geschätzt wird. n-Butan hat dagegen weniger bedeutende Verwendungen gefunden. Es dient z.B. als Butangas zu Heizzwecken oder wird in vergleichsweise kleinen Mengen beispielsweise zur Herstellung von Polymeren oder Copolymeren oder von Maleinsäureanhydrid durch Luftoxidation verwendet. Früher wurde n-Butan auch über die Stufe des n-Butens zu 1.3-Butadien dehydriert, doch ist dieses Verfahren inzwischen unwirtschaftlich geworden.

Weil iso-Butan der begehrtere Bestandteil des Feldbutans ist, wird n-Butan in großem Maßstab zu iso-Butan isomerisiert (vgl. z.B. R.A. Pogliano et al., Dehydrogenation-based Ether Production. 1996 Petrochemical Review, DeWitt & Company, Houston, Texas, Butamer^{(R)}-Verfahren, Seite 6; sowie S.T.Bakas, F.Nierlich et al., Production of Ethers from Field Butanes and Refinery Streams. AIChE Summer Meeting. 1990, San Diego. California, Seite 11). Es lag daher **nicht** im Trend der Technik, ein Verfahren zu entwickeln, das n-Butan als solches nutzt oder gar iso-Butan in n-Butan umwandelt, um daraus mehr Di-n-buten herzustellen.

### (A) Herstellung von Di-n-buten

Das Feldbutan 1 wird zunächst in der Trennstufe **4** in n-Butan **5** und iso-Butan 15 getrennt. Dies geschieht am besten in einer gut wirksamen Kolonne, in der n-Butan 5 durch fraktionierte Destillation bei tiefer Temperatur oder vorteilhaft unter erhöhtem Druck, zweckmäßig von 4 bis 7 bar, von iso-Butan **15** getrennt wird. das je nach Druck um ca. 10 bis 20°C niedriger siedet. Die C_{>4}-Kohlenwasserstoffe fallen dabei als Sumpfprodukt an, n-Butan wird im Seitenstrom abgezogen, und iso-Butan geht zusammen mit leichteren Enden über Kopf.

Das n-Butan **5** wird in die Dehydrierungsstufe 6 geleitet, die für sich gut bekannt ist. Dadurch erhält man ein n-Buten enthaltendes Dehydrierungsgemisch **7**. Hierfür brauchbare Verfahren zur Dehydrierung von leichten Kohlenwasserstoffen sind z.B. von G.C.Sturtevant et al. in Oleflex - Selective Production of Light Olefins, 1988 UOP Technology Conference, sowie in EP 0 149 698 beschrieben. Man führt die Dehydrierung zweckmäßig in der Gasphase an fest angeordneten oder fluidisierten Katalysatoren durch, z.B. an Chrom(III)-oxid oder vorteilhaft an Platinkatalysatoren mit Aluminiumoxid oder Zeolithen als Träger. Die Dehydrierung findet im allgemeinen bei Temperaturen von 400 bis 800°C, vorteilhaft von 550 bis 650°C statt. Man arbeitet in der Regel bei Atmosphärendruck oder leicht erhöhtem Druck bis zu 3 bar. Die Verweilzeit in der Katalysatorschicht liegt, je nach Katalysator, Temperatur und angestrebtem Umsatzgrad, im allgemeinen zwischen 1 und 60 Minuten. Der Durchsatz liegt dementsprechend in der Regel zwischen 0,6 und 36 kg n-Butan je m³ Katalysator und Stunde.

Es ist zweckmäßig, die Dehydrierung nur soweit zu treiben, daß im Dehydrierungsgemisch **7** etwa 50% des n-Butans unverändert bleibt. Zwar kann man bei höherer Temperatur höhere Umsatzgrade erreichen. Dann laufen aber in steigendem Maße Crackreaktionen ab, die die Ausbeute vermindern und, infolge von Koksablagerungen, die Lebensdauer des Dehydrierungskatalysators herabsetzen. Die optimalen Kombinationen der Reaktionsbedingungen, die zu den gewünschten Umsatzgraden führen, wie Art des Katalysators. Temperatur und Verweilzeit. lassen sich unschwer durch orientierende Versuche ermitteln.

Das Dehydrierungsgemisch **7** enthält in der Regel 90 bis 95 % C₄-Kohlenwasserstoffe und daneben Wasserstoff sowie niedriger- und höhersiedende Anteile. Es wird vor der Oligomerisierung zweckmäßig gereinigt. In einer ersten Reinigungsstufe (in der Figur nicht dargestellt) werden die C₄-Fraktion und die höhersiedenden Anteile herauskondensiert. Das Kondensat wird unter Druck destilliert, wobei mitkondensierte, gelöste C_{<4}-Kohlenwasserstoffe über Kopf gehen. Aus dem Sumpfprodukt gewinnt man in einer weiteren Destillation als Hauptprodukt die C₄-Kohlenwasserstoffe und als Rückstand die vergleichsweise kleine Menge an C>₄-Kohlenwasserstoffen.

Die C₄-Kohlenwasserstoffe enthalten im allgemeinen kleinen Mengen, wie 0,01 bis 5 Volumenprozent, 1,3-Butadien. Es ist empfehlenswert, diesen Bestandteil zu entfernen, da er selbst in deutlich geringeren Mengen den Oligomerisierungskatalysator schädigen kann. Ein geeignetes Verfahren ist die Selektivhydrierung **8**, die zudem den Anteil des erwünschten n-Butens erhöht. Ein geeignetes Verfahren wurde z.B. von F.Nierlich et al. in Erdöl & Kohle, Erdgas, Petrochemie. 1986,, Seite 73 ff. beschrieben. Es arbeitet in flüssiger Phase mit vollständig gelöstem Wasserstoff in stöchiometrischen Mengen. Als selektive Hydrierkatalysatoren eignen sich z.B. Nickel und insbesondere Palladium auf einem Träger, z.B. 0,3 Gewichtsprozent Palladium auf Aktivkohle oder vorzugsweise auf Aluminiumoxid. Eine geringe Menge Kohlenmonoxid im ppm-Bereich fördert die Selektivität der Hydrierung des 1.3-Butadiens zum Monoolefin und wirkt der Bildung von Polymeren. dem sogenannten "green oil" entgegen, die den Katalysator inaktivieren. Das Verfahren arbeitet im allgemeinen bei Raumtemperatur oder erhöhter Temperatur bis zu 60°C und unter erhöhten Drücken, die zweckmäßig im Bereich von bis zu 20 bar liegen. Der Gehalt an 1.3-Butadien im Dehydrierungsgemisch wird auf diese Weise auf Werte von <1 ppm gesenkt.

Weiterhin ist es zweckmäßig, die nunmehr von 1,3-Butadien weitgehend befreite C₄-Fraktion des Dehydrierungsgemisches **7** vor der Oligomerisierungsstufe über eine weitere Reinigungsstufe **9**, ein Molekularsieb, zu leiten, wodurch weitere für den Oligomerisierungskatalysator schädliche Stoffe entfernt werden und dessen Lebensdauer weiter erhöht wird. Zu diesen schädlichen Stoffen zählen Sauerstoff- und Schwefelverbindungen. Dieses Reinigungsverfahren ist von F.Nierlich et al. in EP-B1 0 395 857 beschrieben worden. Man verwendet zweckmäßig ein Molekularsieb mit einem Porendurchmesser von 4 bis 15 Angström, vorteilhaft von 7 bis 13 Angström. In manchen Fällen ist es aus wirtschaftlichen Gründen zweckmäßig, das Dehydrierungsgemisch nacheinander über Molekularsiebe mit unterschiedlichen Porengrößen zu leiten. Das Verfahren kann in der Gasphase, in Flüssigphase oder in Gasflüssigphase durchgeführt werden. Der Druck beträgt dementsprechend im allgemeinen 1 bis 200 bar. Man arbeitet zweckmäßig bei Raumtemperatur oder erhöhten Temperaturen bis zu 200 °C.

Die chemische Natur der Molekularsiebe ist weniger wichtig als ihre physikalische Beschaffenheit, d.h. insbesondere die Porengröße. Man kann also die verschiedensten Molekularsiebe einsetzen, sowohl kristalline, natürliche Aluminiumsilikate, z.B. Schichtgittersilikate, als auch synthetische Molekularsiebe, z.B. solche mit Zeolithstruktur. Zeolithe vom A-, X- und Y-Typ sind u.a. von Bayer AG, Dow Chemical Co., Union Carbide Corporation. Laporte Industries Ltd. und Mobil Oil Co. erhältlich. Für das Verfahren eignen sich auch solche synthetische Molekularsiebe, die neben Aluminium und Silicium noch andere, durch Kationenaustausch eingeführte Atome enthalten, wie Gallium, Indium oder Lanthan sowie Nickel, Kobalt. Kupfer, Zink oder Silber. Weiterhin sind synthetische Zeolithe geeignet, bei denen neben Aluminium und Silicium noch andere Atome, wie Bor oder Phosphor, durch Mischfällung in das Gitter eingebaut worden sind.

Wie bereits gesagt, sind die Selektivhydrierungstufe 8 und die Reinigungsstufe **9** mit einem Molekularsieb fakultative, vorteilhafte Maßnahmen für das Verfahren nach der Erfindung. Deren Reihenfolge ist im Prinzip beliebig, jedoch wird die in der Figur angegebene Reihenfolge bevorzugt.

Das gegebenenfalls auf die beschriebene Weise vorbehandelte Dehydrierungsgemisch **7** wird in die Oligomerisierungsstufe 10 geleitet. die ein wesentlicher Teil des Verfahrens nach der Erfindung ist. Die Oligomerisierung wird in an sich bekannter Weise durchgeführt wird, wie z.B. von F. Nierlich in Oligomerization for Better Gasoline, Hydrocarbon Processing, **1992** (2), Seite 45 ff., oder von F.Nierlich et al. in dem bereits erwähnten EP-B1 0 395 857 beschrieben wurde. Man arbeitet im algemeinen in flüssiger Phase und wendet als homogenen Katalysator z.B. ein System an, das aus Nickel-(II)-octoat, Ethylaluminiumchlorid und einer freien Fettsäure besteht (DE-PS 28 55 423), oder benutzt vorzugsweise einen der zahlreichen bekannten, fest angeordneten oder im Oligomerisierungsgemisch suspendierten Katalysatoren auf Basis von Nickel und Silicium. Die Katalysatoren enthalten oftmals zusätzlich Aluminium. So beschreibt die DD-PS 160 037 die Herstellung eines Nickel und Aluminium enthaltenden Fällungskatalysators auf Siliciumdioxid als Trägermaterial. Andere brauchbare Katalysatoren erhält man, indem man auf der Oberfläche der Trägermaterialien befindliche positiv geladene Teilchen, wie Protonen oder Natriumionen, gegen Nickelionen austauscht. Dies gelingt bei den verschiedensten Trägermaterialien. wie amorphem Aluminiumsilikat (R.Espinoza et al., Appl.Kat., **31** (1987), Seiten 259-266; kristallinem Aluminiumsilikat (DE-PS 20 29 624): Zeolithen vom ZSM-Typ (NL-PS 8 500 459); einem X-Zeolith (DE-PS 23 47 235); X-und Y-Zeolithen (A.Barth et al., Z.Anorg-.Allg.Chem. **521**, (1985) Seiten 207-214); und einem Mordenit (EP-A 0 233 302).

Die Oligomerisierung erfolgt zweckmäßig, je nach Katalysator, bei 20 bis 200°C und unter Drücken von 1 bis 100 bar. Die Reaktionszeit (oder Kontaktzeit) beträgt im allgemeinen 5 bis 60 Minuten. Die Verfahrensparameter, insbesondere die Art des Katalysators, die Temperatur und die Kontaktzeit, werden so aufeinander abgestimmt, daß der gewünschte Oligomerisierungsgrad, d.h. vorwiegend eine Dimerisierung, erreicht wird. Dazu darf man die Reaktion natürlich nicht auf vollen Umsatz fahren, sondern strebt zweckmäßig Umsätze von 30 bis 70 % pro Durchgang an. Die optimalen Kombinationen der Verfahrensparameter lassen sich durch orientierende Versuche ohne Schwierigkeiten ermitteln.

Aus dem Oligomerisierungsgemisch **11** werden die Restgase **14** abgetrennt und in die Dehydrierungsstufe **6** zurückgeführt. Wenn in der Oligomerisierungsstufe **10** ein Katalysator vom Typ der erwähnten flüssigen Katalysatoren verwendet wurde, sollten die Restgase **14** zuvor zur Schonung des Dehydrierungskatalysators gereinigt werden. Man behandelt zunächst das Oligomerisierungsgemisch mit Wasser, um die Katalysatorbestandteile zu extrahieren. Das abgetrennte Restgas wird dann mit einem geeigneten Molekularsieb getrocknet, wobei auch andere Nebenbestandteile abgetrennt werden. Danach entfernt man durch selektive Hydrierung, z.B. an Palladiumkatalysatoren, mehrfach ungesättigte Verbindungen, wie Butine, und führt schließlich das so gereinigte Restgas in die Dehydrierungsstufe 6 zurück. Diese Reinigungsmaßnahmen des Restgases erübrigen sich, wenn ein fester Oligomerisierungskatalysator verwendet wird.

Aus der verbleibenden flüssigen Phase des Oligomerisierungsgemisches 11 werden durch fraktionierte Destillation Di-n-buten 12 sowie trimeres n-Buten **13**, d.h. isomere Dodecene, abgetrennt. wobei das Di-n-buten als Hauptprodukt unmittelbar für die Herstellung von Nonanolen geeignet ist. Die Dodecene **13** sind ein erwünschtes Nebenprodukt. Sie können hydroformyliert, die Hydroformylierungsprodukte hydriert und die so erhaltenen Tridecanole oxethyliert werden, wodurch man wertvolle Waschrohstoffe erhält.

### (B) Herstellung von RTBE

Das iso-Butan **15** aus der Trennstufe **4** wird in die Dehydrierungsstufe 16 geleitet und dort zu einem iso-Buten enthaltenden Dehydrierungsgemisch **17** umgesetzt. Hinsichtlich der Verfahrensbedingungen unterscheidet sich diese Dehydrierung nicht wesentlich von derjenigen des n-Butans in der Dehydrierungsstufe **6**. iso-Butan läßt sich leichter dehydrieren als n-Butan, so daß man innerhalb des bei der Dehydrierungsstufe 6 angegebenen Rahmens insgesamt etwas mildere Bedingungen wählen kann. Es ist auch bei dieser Dehydrierung zweckmäßig, nur einen Umsatz von etwa 50 % anzustreben.

Das Dehydrierungsgemisch **17** enthält, wie zuvor für das Dehydrierungsgemisch **7** beschrieben, neben C₄-Kohlenwasserstoffen Wasserstoff sowie leichter siedende Bestandteile (die teilweise aus den Feldbutanen stammen und teilweise bei der Dehydrierung entstehen) sowie höhersiedende Anteile und wird zweckmäßig vor der Veretherung gereinigt. Dies geschieht wiederum in einer (in der Figur ebenfalls nicht dargestellten) ersten Reinigungsstufe, die derjenigen entspricht. die für die Reinigung des Dehydrierungsgemisches **7** beschrieben wurde.

Der so erhaltene C₄-Anteil des Dehydrierungsgemisch **17** wird zweckmäßig über eine Selektivhydrierstufe **18** geleitet, in der Diene, wie Propadien und 1,3-Butadien, selektiv zu Monoolefinen hydriert werden. Die Diene entstehen z.B. aus Propan, das mit den Feldbutanen eingeschleppt wurde, aus n-Butan. das in der Trennstufe **4** nicht vollständig von iso-Butan getrennt wurde, oder entstehen unter den Dehydrierungsbedingungen durch Isomerisierung und/oder Crackreaktionen. Diese Diene stören zumindest bei Rückführung der Restgase **22** die Reaktion in der Dehydrierungsstufe **16**, weniger in der Veretherungsstufe **19**. Die Selektivhydrierungsstufe **18** kann daher auch nach der Veretherungsstufe **19** im Restgasstrom **22** angeordnet sein, vor oder nach der Reinigungsstufe **23**. Diese Anordnung erlaubt es gegebenenfalls. den Reaktor zu verkleinern, weil das Volumen des Restgasstromes **22** natürlich kleiner ist als das des Dehydrierungsgemisches **17**. Hinsichtlich der Verfahrensbedingungen sei auf die Erläuterungen im Zusammenhang mit der Selektivhydrierungsstufe **8** verwiesen.

Das Dehydrierungsgemisch **17** wird, gegebenenfalls nach Selektivhydrierung, in die Veretherungsstufe **19** geleitet, wo das darin enthaltene iso-Buten in an sich bekannter Weise mit einem Alkanol **20** zu einem RTBE umgesetzt wird (siehe z.B. Methyl-Tert-Butyl Ether, Ullmanns Enzyclopedia of Industrial Chemistry, Band A 16. Seiten 543 ff., VCH Verlagsgesellschaft, Weinheim). Von den Alkanolen werden diejenigen mit 1 bis 6 Kohlenstoffatomen bevorzugt, beispielsweise Ethanol, Isopropanol, Isobutanol und insbesondere Methanol. Die Reaktion findet in Flüssigphase oder in Gasflüssigphase bei einer Temperatur von 50 bis 90°C und unter einem Druck statt, der sich bei der jeweiligen Temperatur einstellt. Zweckmäßig arbeitet man mit einem geringen Überschuß an Alkanol, wodurch die Selektivität der Umsetzung des iso-Butens erhöht und dessen Dimerisierung zurückgedrängt wird. Als Katalysator wird z.B. ein saurer Bentonit oder vorteilhaft ein großporiger saurer Ionenaustauscher verwendet.

Aus dem Reaktionsgemisch der Veretherungsstufe **19** trennt man durch Destillation das Restgas **22** und gegebenenfalls überschüssiges Alkanol **20** von dem gebildeten RTBE **21** ab. Im Falle von MTBE bildet das Restgas **22** mit Methanol ein Azeotrop. Das Azeotrop wird mit Wasser gewaschen und in eine wässerige Phase und Restgas **22** getrennt, das in die Dehydrierstufe **16** zurückgeführt wird, gegebenenfalls über die (dann im Verfahrensgang entsprechend angeordnete) Selektivhydrierungsstufe 18 und/oder die Reinigungsstufe **23**, letztere wiederum zweckmäßig eine Behandlung mit einem Molekularsieb, durch die insbesondere sauerstoff- oder schwefelhaltige Verunreinigungen entfernt werden, die den Dehydrierungskatalysator stören. Zumindest ein Teil des Restgases **22** kann auch in die Trennstufe **4** zurückgeführt werden (in der Figur nicht dargestellt), um eine Anreicherung von n-Butan infolge unscharfer n-Buten/iso-Butan-Trennung in der Trennstufe **4** zu vermeiden. Die wässerige Phase, die bei der Wasserwäsche anfällt, wird auf Methanol, das in die Veretherung zurückgeführt wird, und auf Wasser aufgearbeitet, das wieder zur Wäsche benutzt wird.

### (C) Variation der Di-n-buten- und RTBE-Mengen

Es ist zweckmäßig, der Trennstufe **4** eine Isomerisierungsstufe **3** zuzuordnen, weil man dadurch das Verhältnis der Mengen an Di-n-buten und RTBE variieren kann. Die Variationsmöglichkeiten sind nur durch die Kapazitäten der Di-n-buten- und der RTBE-Anlage begrenzt. Mit Rücksicht auf die Investitionskosten wird man wohl selten beide Anlagen so groß auslegen, daß der gesamte zur Verfügung stehende Feldbutan-Strom in nur einer der Anlagen verarbeitet werden kann, während die andere Anlage stilliegt. Trotzdem bringt die Isomerisierungsstufe **3** die Möglichkeit, flexibel auf die Anforderungen des Marktes zu reagieren.

Wenn die Feldbutane **1** ungesättigte Verbindungen enthalten, ist es zweckmäßig, neben der Isomerisierungsstufe **3** eine Hydrierstufe **2** vorzusehen, in der diese ungesättigten Verbindungen hydriert werden, da sie die Isomerisierung stören. Die Hydrierung erfolgt in sich bekannter Weise (siehe z.B. K.H.Walter et al. in The Hüls Process for Selective Hydrogenation of Butadiene in Crude C₄'s, Development and Technical Application, DGKM-Tagung. Kassel. November 1993). Man arbeitet also zweckmäßig in flüssiger Phase und, je nach Katalysator, bei Raumtemperatur oder erhöhter Temperatur bis zu 90°C und unter einem Druck von 4 bis 20 bar, wobei der Partialdruck des Wasserstoffs 1 bis 15 bar beträgt. Man verwendet die für die Hydrierung von Olefinen üblichen Katalysatoren, z.B. 0,3 % Palladium auf Aluminiumoxid.

Die hydrierten Feldbutane **1** werden in die Trennstufe **4** geleitet und dort, wie beschrieben, in n-Butan **5** und iso-Butan **15** getrennt. Soll das n/iso-Verhältnis dem jeweiligen Bedarf der beiden Anlagen entsprechend verändert werden, so zieht man einen Teil des im Überschuß vorhandenen Isomeren in die Isomerisierungsstufe **3** ab. Die alternativen Möglichkeiten sind in der Figur durch gestrichelte Linien angedeutet. In der Isomerisierungsstufe **3** wird das abgezogene Isomere maximal bis zum Gleichgewicht, das je nach der Temperatur bei 40 bis 55 % n-Butan und 45 bis 60 % iso-Butan liegt, in das andere Isomere umgewandelt. Die Isomerisierung von n- und iso-Butan ist eine bekannte Reaktion. Man arbeitet im allgemeinen in der Gasphase bei einer Temperatur von 150 bis 230°C, unter einem Druck von 14 bis 30 bar und mit einem Platin-Katalysator auf Aluminiumoxid als Träger, dessen Selektivität durch Dotierung mit einer Chlorverbindung, wie Tetrachlorkohlenstoff, noch verbessert werden kann. Man setzt vorteilhaft eine kleine Menge Wasserstoff zu, um einer Dehydrierung entgegenzuwirken. Die Selektivität der Isomerisierung ist hoch. Crackung zu kleineren Bruchstücken findet nur in untergeordnetem Maße (ca. 2 %) statt (siehe z.B. H.W.Grote. Oil and Gas Journal, 56 (13). Seite 573 ff. (1958)).

Das Isomerisierungsgemisch **24** muß in die Isomeren getrennt werden und wird dazu zweckmäßig in die ohnehin vorhandenen Trennstufe **4** geleitet.

## Patentansprüche

1. Verfahren zur Herstellung von Di-n-butan und Alkyl-tert.butylethern in einer Koppelproduktion aus Feldbutanen, dadurch gekennzeichnet, daß man
(a) die Feldbutane **1** in der Trennstufe **4** in n-Butan und iso-Butan trennt,
(b) das n-Butan **5** in einer Dehydrierungsstufe **6** zu einem n-Buten enthaltenden Dehydrierungsgemisch **7** dehydriert, das n-Buten in der Oligomerisierungsstufe **10** zu einem Oligomerengemisch **11** oligomerisiert und aus diesem Di-n-buten **12** abtrennt, und
(c) das iso-Butan **15** in der Dehydrierungsstufe **16** zu einem iso-Buten enthaltenden Dehydrierungsgemisch **17** dehydriert und das iso-Buten in der Veretherungsstufe **19** mit einem Alkanol **20** zu einem Alkyl-tert.butylether **21** umsetzt.

2. Verfahren nach Anspruch 1, dadurch gekennzeichnet, daß man die Feldbutane **1** vor dem Eintritt in die Trennstufe **4** in der Hydrierstufe **2** Hydrierungsbedingungen unterwirft und der Trennstufe **4** eine Isomerisierungsstufe **3** zuordnet, durch die das Mengenverhältnis von n-Butan zu iso-Butan nach Maßgabe des gewünschten Mengenverhältnisses von Alkyl-tert.butylether zu Di-n-buten eingestellt wird.

3. Verfahren nach Anspruch 1 oder 2, dadurch gekennzeichnet, daß man zwischen der Dehydrierungsstufe **6** und der Oligomerisierungsstufe **10** eine Selektivhydrierung **8** und/oder eine Reinigungsstufe **9** in beliebiger Reihenfolge anordnet.

4. Verfahren nach einem der Ansprüche 1 bis 3, dadurch gekennzeichnet, daß man aus dem Oligomerisierungsgemisch **11** die Restgase **14** abtrennt und diese, gegebenenfalls nach Reinigung, in die Dehydrierungsstufe 6 zurückführt.

5. Verfahren nach einem der Ansprüche 1 bis 4, dadurch gekennzeichnet, daß man zwischen der Dehydrierungsstufe **16** und der Veretherungsstufe 19 eine Selektivhydrierstufe **18** anordnet.

6. Verfahren nach einem der Ansprüche 1 bis 5, dadurch gekennzeichnet, daß man die Restgase **22** aus der Veretherungsstufe **19** über eine Reinigungsstufe **23** in die Dehydrierungsstufe **16** zurückführt.

7. Verfahren nach einem der Ansprüche 1 bis 6, dadurch gekennzeichnet, daß man als Alkanol Ethanol, Isopropanol, Isobutanol oder insbesondere Methanol verwendet.

## Claims

1. A process for preparing di-n-butane and alkyl tert-butyl ethers in a coupled production from field butanes, which comprises
(a) separating the field butanes 1 into n-butane and isobutane in the separation stage 4,
(b) dehydrogenating the n-butane 5 in a dehydrogenation stage 6 to give an n-butene-containing dehydrogenation mixture 7, oligomerizing the n-butene in the oligomerization stage 10 to give an oligomer mixture 11 and separating di-n-butene 12 off from this, and
(c) dehydrogenating the isobutane 15 in the dehydrogenation stage 16 to give an isobutene-containing dehydrogenation mixture 17 and reacting the isobutene with an alkanol 20 in the etherification stage 19 to give an alkyl tert-butyl ether 21.

2. A process according to claim 1, characterized in that the field butanes 1, prior to entry into the separation stage 4, are subjected to hydrogenation conditions in the hydrogenation stage 2 and an isomerization stage 3 is assigned to the separation stage 4, by means of which isomerization stage the ratio of n-butane to isobutane is set in accordance with the desired ratio of alkyl tert-butyl ether to di-n-butene.

3. A process according to claim 1 or 2, characterized in that a selective hydrogenation 8 and/or a purification stage 9, in any order, is arranged between the dehydrogenation stage 6 and the oligomerization stage 10.

4. A process according to any one of claims 1 to 3, characterized in that the residual gasses 14 are separated off from the oligomerization mixture 11 and these residual gasses, if appropriate after purification, are recycled to the dehydrogenation stage 6.

5. A process according to any one of claims 1 to 4, characterized in that a selective hydrogenation stage 18 is arranged between the dehydrogenation stage 16 and the etherification stage 19.

6. A process according to any one of claims 1 to 5, characterized in that the residual gasses 22 from the etherification stage 19 are recycled via a purification stage 23 to the dehydrogenation stage 16.

7. A process according to any one of claims 1 to 6, characterized in that the alkanol used is ethanol, isopropanol, isobutanol or, in particular, methanol.

## Revendications

1. Procédé de préparation de di-n-butane et d'éthers d'alkyle et de terbutyle dans une production couplée à partir de butanes de terrain
caractérisé en ce qu'
a) on sépare les butanes de terrain (1) dans l'étage de séparation (4) en n-butane et en isobutane,
b) le n-butane (5) est deshydrogèné dans un étage de déshydrogénation (6) en un mélange de déshydrogénation contenant du n-butène (7), on oligomérise le n-butène dans l'étage d'oligomérisation (10) en un mélange d'oligomères (11), et on sépare de celui-ci le di-n-butène (12), et
c) l'isobutane (15) est deshydrogèné dans l'étage de déshydrogénation (16) en un mélange de déshydrogénation contenant de l'isobutène (17), et on fait réagir l'isobutène dans l'étage d'éthérification (19) avec un alcanol (20) en un éther d'alkyle et de terbutyle (21).

2. Procédé selon la revendication 1,
caractérisé en ce qu'
on soumet les butanes de terrain (1) avant l'entrée dans l'étage de séparation (4) dans l'étage de déshydrogénation (2) aux conditions d'hydrogénation, et on adjoint à l'étage de séparation (4) un étage d'isomérisation (3), par lequel le rapport en quantité du butane à l'isobutane est ajusté selon la proportion du rapport désiré en quantité d'éther d'alkyle et de terbutyle au di-n-butène.

3. Procédé selon la revendication 1 ou 2,
caractérisé en ce qu'
on dispose entre l'étage de déshydrogénation (6) et l'étage d'oligomérisation (10) une hydrogénation sélective (8) et/ou un étage de purification (9) en série au choix.

4. Procédé selon l'une quelconque des revendications 1 à 3,
caractérisé en qu'
on sépare du mélange d'oligomérisation (11) les gaz résiduels (14) et on recycle ceux-ci dans l'étage de déshydrogénation (6), éventuellement après purification.

5. Procédé selon l'une quelconque des revendications 1 à 5,
caractérisé en ce qu'
on dispose entre l'étage de déshydrogénation (16) et l'étage d'éthérification (19) un étage d'hydrogénation sélective.

6. Procédé selon l'une quelconque des revendications 1 à 5,
caractérisé en ce qu'
on recycle les gaz résiduels (22) provenant de l'étage d'éthérification (19) dans l'étage de déshydrogénation (16) par l'intermédiaire d'un étage de purification (23).

7. Procédé selon l'une quelconque des revendications 1 à 6,
caractérisé en ce qu'
on utilise comme alcanol de l'éthanol, de l'isopropanol, de l'isobutanol ou en particulier du méthanol.
